# EUROPÄISCHE PATENTSCHRIFT

(11) **EP 2 148 855 B1**
(45) Veröffentlichungstag und Bekanntmachung des Hinweises auf die Patenterteilung: **02.10.2013**
(21) Anmeldenummer: 08736655.5
(22) Anmeldetag: 06.05.2008
(51) Int. Cl.: C07C 321/04, C07C 319/02

(54) **VERFAHREN ZUR KONTINUIERLICHEN HERSTELLUNG VON METHYLMERCAPTAN AUS KOHLENSTOFF- UND WASSERSTOFFHALTIGEN VERBINDUNGEN**
PROCESS FOR CONTINUOUSLY PREPARING METHYL MERCAPTAN FROM CARBON- AND HYDROGEN-CONTAINING COMPOUNDS
PROCÉDÉ DE PRODUCTION EN CONTINU DE MÉTHYLMERCAPTAN À PARTIR DE COMPOSÉS CONTENANT DU CARBONE ET DE L'HYDROGÈNE

(30) Priorität: 25.05.2007 DE 102007024576
(43) Veröffentlichungstag der Anmeldung: 03.02.2010
(73) Patentinhaber: Evonik Degussa GmbH, 45128 Essen (DE)
(72) Erfinder: BARTH, Jan-Olaf, 60598 Frankfurt (DE); REDLINGSHÖFER, Hubert, 91481 Münchsteinach (DE); FINKELDEI, Caspar-Heinrich, 63755 Alzenau (DE); WECKBECKER, Christoph, 63584 Gründau-lieblos (DE); HUTHMACHER, Klaus, 63571 Gelnhausen (DE)
(86) Internationale Anmeldenummer: PCT/EP2008/055548
(87) Internationale Veröffentlichungsnummer: WO 2008/145488

(56) Entgegenhaltungen:
- WO-A-00/56692
- WO-A-01/96290
- WO-A-2005/040082
- US-A- 4 410 731
- US-A- 4 665 242

## Beschreibung

Die Erfindung betrifft ein Verfahren zur kontinuierlichen Herstellung von Methylmercaptan durch Umsetzen eines Eduktgemisches das feste, flüssige und/oder gasförmige kohlenstoff- und wasserstoffhaltige Verbindungen enthält, mit Luft oder Sauerstoff und/oder Wasser und Schwefel.

Methylmercaptan ist ein industriell wichtiges Zwischenprodukt für die Synthese von Methionin sowie für die Herstellung von Dimethylsulfoxid und Dimethylsulfon. Methylmercaptan wird überwiegend aus Methanol und Schwefelwasserstoff durch Reaktion an einem Katalysator, bestehend aus einem Aluminiumoxidträger und Übergangsmetalloxiden und basischen Promotoren hergestellt. Die Synthese des Mercaptans erfolgt gewöhnlich in der Gasphase bei Temperaturen zwischen 300 und 500°C und bei Drücken zwischen 1 und 25 bar. Das Produktgasgemisch enthält neben dem gebildeten Methylmercaptan und Wasser die nicht umgesetzte Anteile der Ausgangsstoffe Methanol und Schwefelwasserstoff und als Nebenprodukte Dimethylsulfid und Dimethylether, sowie in geringen Mengen auch Polysulfide (Dimethyldisulfid). Im Sinne der Reaktion inerte Gase wie beispielsweise Kohlenmonoxid, Kohlendioxid, Stickstoff und Wasserstoff sind auch im Produktgas enthalten.

Aus dem Produktgasgemisch wird das gebildete Methylmercaptan, wie z. B. in der US 5866721 erläutert, in mehreren Destillations- und Waschkolonnen bei Temperaturen zwischen 10 und 140°C abgetrennt.

Methylmercaptan kann alternativ aus Kohlenstoffoxiden, Wasserstoff, Schwefel und/oder Schwefelwasserstoff hergestellt werden. Gemäss US 4665242 erfolgt beispielsweise die Darstellung von Methylmercaptan über Katalysatoren auf der Basis von Alkalimetallwolframaten. Im Vergleich zum methanolbasierten Verfahren weisen diese Prozesse niedrigere Selektivitäten für Methylmercaptan und Umsätze an Kohlenstoffoxiden auf. Die US 4410731 betrifft ein Verfahren und Katalysatoren für die Darstellung von Methylmercaptan aus Kohlenstoffoxiden, Wasserstoff und Schwefelwasserstoff oder Schwefel auf der Basis von mit Übergangsmetalloxiden als Promotoren enthaltenden Alkalimetallmolybdänsulfiden und Aluminiumoxid als Träger. In der WO2005/040082 werden ein Verfahren und Katalysatoren für die Darstellung von Methylmercaptan aus Kohlenstoffoxiden, Wasserstoff und Schwefelwasserstoff oder Schwefel auf der Basis von Übergangsmetalloxide als Promotoren enthaltenden Alkalimetallmolybdaten beansprucht, bei denen vorzugsweise Siliziumdioxid als Träger dient.

Die Darstellung von Methylmercaptan aus Schwefelkohlenstoff oder Carbonylsulfid und Wasserstoff ist eine weitere Alternative zum auf Methanol basierenden Verfahren. Die Prozesse sind jedoch charakterisiert durch vergleichsweise niedrige Selektivitäten für Methylmercaptan, eine Vielzahl von Nebenprodukten, die aufwendig und kostenintensiv abzutrennen sind, sowie die Notwendigkeit, toxischen Schwefelkohlenstoff bzw. Carbonylsulfid in großen Mengen handzuhaben.

Die direkte Umsetzung von bei anderen Prozessen anfallenden Gemischen, die Methan oder höhere Kohlenwasserstoffe, Wasser, Wasserstoff und gegebenenfalls schwefelhaltige Verbindungen enthalten, zu Methylmercaptan, gelang bis dato nicht in technisch relevanten Ausbeuten und Selektivitäten, sondern führt zu einer Vielzahl von Nebenprodukten mit u.a. toxischem Schwefelkohlenstoff als Hauptkomponente.

Allen oben genannten Verfahren ist gemeinsam, dass für die Darstellung von Methylmercaptan Kohlenstoffverbindungen wie Methanol, Kohlenoxide oder Schwefel-haltige wie Carbonylsulfid oder Schwefelkohlenstoff als Rohstoffe benötigt werden. Diese stellen einen signifikanten Kostenfaktor dar, besonders, wenn die Produktselektivitäten für die Bildung von Methylmercaptan vergleichsweise gering sind. Ferner sind teils aufwendige Reinigungsverfahren notwendig, bei denen eine Vielzahl von Nebenkomponenten nicht in den Prozess zurückgeführt werden können. Das verringert die Gesamtselektivität für Methylmercaptan und somit die Wirtschaftlichkeit des Verfahrens.

Die direkte Umsetzung von Edukten, die Erdgas (Methan), Kohlenwasserstoffe z. B. aus Schwer-ölfraktionen, Rückständen aus der Erdölraffination oder allgemein höheren Kohlenwasserstoffen, wie z.B. Oligomere, Polymere oder polycyclische Aromaten , die normalerweise z. B. auch als Abfallströme in anderen chemischen Prozessen anfallen, mit Luft oder Sauerstoff, Wasser und Schwefel zu Methylmercaptan ist Aufgabe der Erfindung. Sie weist gegenüber dem technisch praktizierten, methanolbasierten Verfahren, aufgrund deutlich niedrigerer Rohstoffkosten, einen signifikanten Kostenvorteil in den variablen Betriebskosten auf. Wie aus dem Vorgenannten für den Fachmann ersichtlich, steht nach dem Stand der Technik ein solches Verfahren nicht zur Verfügung. Bedingt durch vergleichsweise niedrige Produktselektivitäten und ein breites Spektrum von Nebenprodukten, sowie die Toxizität der Reaktionsintermediate, wodurch umfangreiche Sicherheitsmaßnahmen zum Schutz von Mensch und Umwelt notwendig werden, existierte ein derartiges Verfahren zur Darstellung von Methylmercaptan nicht.

Die Wirtschaftlichkeit des Gesamtprozesses hängt entscheidend von der Produktselektivität für Methylmercaptan, bezogen auf die eingesetzte Kohlenstoffquelle, ab. Durch den erfindungsgemäßen Einsatz von kohlenstoff- und wasserstoffhaltigen Verbindungen, die in anderen Prozessen als Nebenkomponenten bzw. als Abfallströme anfallen oder nur als Brennstoff zur Energieerzeugung genutzt werden, kann ein zusätzlicher Kostenvorteil erzielt werden. Beispielsweise fallen bei selektiven Oxidationen eine Reihe von Nebenkomponenten an, die zusätzlich chemisch gebundenem Sauerstoff enthalten, wie z.B. Alkohole, Aldehyde, Carbonsäuren und/oder Kohlenoxide. Diese werden in der Regel nach der Abtrennung des Hauptreaktionsproduktes verbrannt oder in einer weiteren chemischen Reaktion so umgesetzt, dass eine Entsorgung möglich ist. Weitere Beispiele für Quellen von Eduktgemischen sind technische Verfahren zur Erzeugung von organischen Stickstoff- oder Schwefelverbindungen, in denen größere Mengen von Nebenprodukten anfallen, die in der Regel ohne weitere Wertschöpfung verbrannt oder andersweitig entsorgt werden müssen. Insbesondere können in dem erfindungsgemäßen Verfahren Abgasströme, die H₂S, COS, SO₂, SO₃-haltige Verbindungen, Alkylsulfide oder Alkylpolysulfide enthalten, eingesetzt werden. Hierzu zählen explizit auch Gase, die aus Abgasströmen von Anlagen zur Erzeugung von Energie oder chemischen Produkten direkt oder über Separationstechniken gewonnen werden, bzw. im Rahmen von biologischen Stoffwechselprozessen (z.B. Fermentations- und Abbauprozesse) entstehen. Diese Gasmischungen können als Hauptkomponenten Kohlenwasserstoffe, Kohlenstoffoxide, organische Schwefel- und Stickstoffverbindungen oder Schwefelwasserstoff neben anderen Stoffen enthalten und dem erfindungsgemäßen Verfahren zugeführt werden.

Aufgabe der Erfindung ist die Bereitstellung eines wirtschaftlichen Verfahrens zur Herstellung von Methylmercaptan aus Eduktmischungen, die kohlenstoff- und wasserstoffhaltige Verbindungen enthalten, Luft oder Sauerstoff, und/oder Wasser und Schwefel.

Gegenstand der Erfindung ist ein Verfahren zur kontinuierlichen Herstellung von Methylmercaptan durch Umsetzung eines Eduktgemisches, das kohlenstoff- und wasserstoffhaltige Verbindungen enthält, in einem mehrstufigen Verfahren, bei dem man in Verfahrensschritt 1 die kohlenstoff- und wasserstoffhaltigen Verbindungen mit Luft oder Sauerstoff gegebenenfalls mit Wasser, zu einer Gasmischung umsetzt, die Kohlendioxid, Kohlenmonoxid und Wasserstoff als Hauptkomponenten enthält.

Die kohlenstoffhaltigen Verbindungen können in festem, flüssigen oder gasförmigem Zustand bereitgestellt werden, liegen aber zum Zeitpunkt der Umsetzung bevorzugt gasförmig vor.

Daneben kann das Eduktgas organische Schwefelverbindungen oder H₂S enthalten.

CO, CO₂ und Wasserstoff sind in dem aus Verfahrensschritt 1 austretenden Gasgemisch im allgemeinen in einer Menge von 1 bis 90 Vol% enthalten.

Bevorzugt sind Konzentrationen von 5 bis 25 Vol% für CO., 5 bis 50 Vol% für CO₂ und von 10 bis 90 Vol% für H₂, wobei die Gesamtmenge bevorzugt < 100 Vol%, insbesondere bei ∼ 90 Vol% bleibt..

Anschließend erfolgt die direkte Umsetzung dieser Gasmischung ohne weitere Kompression und Aufarbeitung der Gase bei einem Reaktionsdruck von mindestens 5 bar und einer Temperatur von mindestens 200°C mit flüssigem oder gasförmigen Schwefel in einem ein- oder mehrstufigen Verfahrensschritt 2..

Das molare CO₂/CO/H₂/H₂S-Verhältnis wird durch Einspeisen von Wasser oder Wasserstof und gegebenenfalls Schwefelwasserstoff auf ein Verhältnis von 1:0,1:1:0 bis 1:1:10:10 eingestellt.

In Verfahrensschritt 3 wird diese Gasmischung bei einem Reaktionsdruck von mindestens 5 bar und einer Temperatur von mindestens 200°C über einem Katalysator zu einer Reaktionsmischung umgesetzt, die als Hauptprodukt Methylmercaptan enthält.

Anschließend erfolgt die Abtrennung des Methylmercaptans mit an sich bekannten Verfahren in Verfahrensschritt 4.

In Verfahrensschritt 5 werden nach Abtrennung der gasförmigen Nebenprodukte die nicht umgesetzten Einsatzstoffe nach optionaler Umsetzung mit Wasser in den Prozess zurückgeführt.

Den ersten Verfahrensschritt verlässt die Gasmischung bei einem Druck von mindestens 5 bar und wird direkt ohne weitere Kompression dem zweiten, und in der Folge die weiteren Gasmischungen bei diesem Druck dem dritten und vierten Verfahrensschritt zugeführt..

Die Gesamtselektivität für Methylmercaptan kann durch Recyclierung von kohlenstoff-, wasserstoff- und schwefelhaltigen Verbindungen in den ersten, zweiten oder dritten Verfahrensschritt erhöht werden.. Vorzugsweise werden Kohlenstoffoxide, Wasserstoff, Carbonylsulfid und Schwefelwasserstoff in den zweiten oder dritten Verfahrensschritt recycliert, während Nebenprodukte, wie z.B. Wasser, Kohlenwasserstoffe und andere schwefelhaltige Verbindungen, wie z.B. (Poly-) Sulfide und Schwefelkohlenstoff in den ersten Verfahrensschritt zurückgeführt werden. Ein besonderer Vorteil der Erfindung ist, dass (Poly-) Sulfide und toxischer Schwefelkohlenstoff mit Selektivitäten kleiner 1 % anfallen und bedingt durch die Recyclierung in das Verfahren, nicht technisch aufwendig und kostenintensiv separiert und entsorgt werden müssen.

Das Produktgasgemisch des dritten Verfahrensschrittes enthält neben dem gebildeten Methylmercaptan und Wasser, die nicht umgesetzten Ausgangsstoffe Kohlendioxid, Kohlenmonoxid, Wasserstoff und Schwefelwasserstoff und als Nebenprodukte Carbonylsulfid, Methan, Dimethylsulfid und in geringen Mengen auch Polysulfide (Dimethyldisulfid) und Schwefelkohlenstoff. Im Sinne der Reaktion inerte Gase wie beispielsweise Stickstoff und Kohlenwasserstoffe sind auch im Produktgas enthalten.

Aus dem Produktgasgemisch wird das gebildete Methylmercaptan, z. B. wie in DE-A-1768826 erläutert, in mehreren Destillations- und Waschkolonnen bei Temperaturen zwischen 10 und 140°C abgetrennt. Kohlendioxid, Kohlenmonoxid, Wasserstoff, Schwefelwasserstoff und als Nebenprodukte Carbonylsulfid, Methan, Dimethylsulfid und in geringen Mengen auch Polysulfide (Dimethyldisulfid) und Schwefelkohlenstoff werden in den ersten, zweiten oder dritten Verfahrensschritt zurückgeführt. Vorteilhafterweise wird der Stoffstrom in einem optionalen, fünften Verfahrensschritt bevorzugt katalytisch mit Wasser in der Weise umgesetzt, dass das in den Prozess zurückgeführte Kreisgas nur noch die Hauptkomponenten CO₂, CO, H₂ und H₂S enthält.

Die Wirtschaftlichkeit des Gesamtprozesses wird dadurch erhöht, dass vorteilhafterweise vor der Dosierung der Einsatzstoffe in den ersten und zweiten Verfahrensschritt keine aufwendige und kostenintensive Abtrennung von potentiellen Katalysatorgiften, wie z.B. schwefelhaltigen Verbindungen und elementarem Schwefel notwendig ist. Ebenso entfällt eine Abtrennung solcher Verbindungen nach der Umsetzung in der ersten Verfahrensstufe. Diese Stoffe können zusammen mit den Reaktionsgasen, ohne weitere Aufarbeitung und Kompression der Gase, direkt dem zweiten Verfahrensschritt zugeführt werden, was einen signifikanten Kostenvorteil hinsichtlich der Investitions- und Betriebskosten des Prozesses darstellt. Es entfällt somit eine kostenintensive Entschwefelung des Edukt- und Produktgemisches des ersten Verfahrensschrittes. Vorteilhafterweise können Schwefel oder schwefelhaltige Schlacken, die gegebenenfalls als Nebenprodukte des Verfahrensschrittes 1 anfallen, direkt in fester, flüssiger oder gasförmiger Form als Edukt dem Verfahrensschritt 2 zugeführt werden. Hierzu zählen explizit auch Gase, die aus Abgasströmen von Anlagen zur Erzeugung von Energie oder chemischen Produkten direkt oder über Separationstechniken gewonnen werden, bzw. im Rahmen von biologischen Abbau- und Stoffwechselprozessen entstehen und direkt dem zweiten Verfahrensschritt zugeführt werden können. Diese Gasmischungen können als Hauptkomponenten Kohlenwasserstoffe, Kohlenstoffoxide, Schwefel- und Stickstoffverbindungen in einer Gesamtkonzentration von 5 bis 90 Vol% neben anderen Stoffen enthalten und den Verfahrensschritten 1, 2 oder 3 zugeführt werden.

Die Umsetzung der Gasmischung in der zweiten Verfahrensstufe kann optional unter Verwendung eines Katalysators durch Reaktion mit flüssigen oder gasförmigen Schwefel in einem ein- oder mehrstufigen Verfahrensschritt erfolgen. In dem zweiten Verfahrensschritt wird kein Vollumsatz an Wasserstoff angestrebt. Die Reaktion wird so durchgeführt, dass nach der Umsetzung das CO₂ / CO / H₂ / H₂S-Verhältnis 1 : 0,1 : 1 : 1 bis 1 : 1 : 10 : 10 beträgt. Vorteilhafterweise verlässt das Reaktionsgas aus dem ersten Verfahrensschritt diesen bei einem Druck von mindestens 5 bar und kann direkt ohne weitere Kompression dem zweiten. Verfahrensschritt zugeführt werden. Dies stellt einen signifikanten Kostenvorteil dar, da auf eine Verdichterstufe mit hohen Investitions- und Betriebskosten verzichtet werden kann. Die Reaktionsgasmischung aus dem zweiten Verfahrensschritt wird anschließend ohne weitere Kompression und Aufarbeitung von Reaktionsprodukten der dritten Verfahrensstufe zugeführt. Optional können Vorrichtungen zur Abtrennung von elementarem Schwefel oder schwefelhaltigen Verbindungen diesem Verfahrensschritt vorgeschaltet sein. Die Umsetzung zu Methylmercaptan erfolgt in einem dritten Verfahrensschritt über Katalysatoren. Als vorteilhaft haben sich im zweiten und dritten Verfahrensschritt Metalloxidkatalysatoren erwiesen. Vorzugsweise werden Katalysatoren auf Basis von Alkalimolybdaten oder Alkaliwolframaten verwendet, die auf Träger aufgebracht sein können (US 5852219). Insbesondere geeignet sind Trägerkatalysatoren, die oxidische Mo- und K-Verbindungen enthalten, wobei Mo und K in einer Verbindung enthalten sein können, wie z. B. K₂MoO₄, und mindestens eine aktive oxidische Verbindung der allgemeinen Formel AₓO_{y} enthalten. Dabei bedeutet A ein Element der Mangangruppe, insbesondere Mn oder Re, und x und y ganze Zahlen von 1 bis 7. Der Katalysator enthält die Verbindungen bevorzugt in einem Gewichtsverhältnis von AₓO_{y}/K₂MoO₄/Träger = 0,001-0,5)/(0,01-0,8)/1 bzw. AₓO_{y}/MoO_{3/}K₂O/Träger [0,0001-0,5)/[0,01-0,8)/[0,005-0,5)/1 wobei die Gewichtsverhältnisse bevorzugt im Bereich AₓO_{y}/K₂MoO_{4/}Träger = (0,001-0,3)/(0,05-0,5)/1 bzw. AₓO₄/MoO₃/K₂O/Träger = [0,001-0,3)/(0,05-0,3)/0,03-0,3/1 liegen.

Diese Katalysatoren enthalten bevorzugt einen oder mehrere Promotoren, ausgewählt aus der Gruppe der oxidischen Verbindungen mit der allgemeinen Formel MₓO_{y}, in denen M für ein Übergangselement oder ein Metall aus der Gruppe der Seltenen Erden steht und x und y eine ganze Zahl von 1 bis 7 bedeuten, entsprechend dem Oxidationsgrad der eingesetzten Elemente M.

Bevorzugt bedeutet M Fe, Co, Ni, La oder Ce. In einer besonderen Ausführungsform kann M auch Sn bedeuten. Die Verhältnisse der Gewichtsanteile liegen in den Bereichen:

K₂MoO_{4/}MₓO_{y}/Träger = (0,01-0,80(/(0,01-0,1)/1,

MoO₃/K₂O/MₓO_{y}/Träger = (0,10-0,50)/(0,10-0,30)/(0,01-0,1)/1,

Werden diese Katalysatoren vor dem Einsatz einer H₂S-haltigen Atmosphäre ausgesetzt, wandeln sich die oxidischen Metallverbindungen, mit denen nicht das Trägermaterial gemeint ist in sulfidische Verbindungen oder Mischungen aus oxidischen und sulfidischen Verbindungen um, die ebenso erfindungsgemäß einsetzbar sind.

Als Trägermaterialien werden bevorzugt Siliciumdioxide, Titandioxide, Zeolithe oder Aktivkohlen eingesetzt. Wenn als Träger Aluminiumoxid verwendet wird, enthält der Katalysator Rheniumoxide und/oder Rheniumsulfid(e).

Titandioxid wird bevorzugt mit einem Gehalt von 60 Mol% Anatas eingesetzt.

Die Herstellung erfolgt in einem mehrstufigen Imprägnierverfahren, mit dem lösliche Verbindungender gewünschten Promotoren oder aktiven oxidischen Verbindungen auf den Träger aufgebracht werden. Der imprägnierte Träger wird anschließend getrocknet und gegebenenfalls calciniert.

Vorzugsweise werden der zweite und dritte Verfahrensschritt in einem Reaktionsapparat kombiniert. Dies kann unter Verwendung von unterschiedlichen oder gleichen Katalysatoren geschehen. Vorteilhafterweise kommen Blasensäulen, Reaktivdestillationen, Festbett-, Horden- oder Rohrbündelreaktoren für die katalysierte Umsetzung zu Methylmercaptan zum Einsatz.

Die Umsetzung im zweiten Verfahrensschritt erfolgt bei einer Temperatur von 200 bis 600°C, insbesondere 250 bis 500°C und einem Druck von 1,5 bis 50 bar. Bevorzugt wird ein Betriebsdruck von 2,5 bis 40 bar eingestellt. Die Umsetzung zu Methylmercaptan erfolgt im dritten Verfahrensschritt über Katalysatoren auf Basis von Alkalimolybdaten oder Alkaliwolframaten. Bei einer Temperatur von 200 bis 600°C, vorzugsweise 250 bis 400°C und einem Druck von 1,5 bis 50 bar, vorzugsweise 8 bis 40 bar. Katalysatoren, die vorteilhafterweise im zweiten und dritten Verfahrensschritt zum Einsatz kommen, werden in den Anmeldungen WO 2005/040082 , WO 2005/021491, WO 2006/015668 und WO 2006/063669 beschrieben.

In einer weiteren Ausführungsform der Erfindung werden der zweite und dritte Verfahrensschritt in einer Vorrichtung kombiniert.

Die Auftrennung des Produktgasgemisches kann nach verschiedenen, bekannten Verfahren erfolgen. Eine besonders vorteilhafte Auftrennung wird in den Patentschriften EP-B-0850923 (US 5866721) beschrieben.

Nicht umgesetzte Kohlenstoffoxide, Wasserstoff und Schwefelwasserstoff, sowie gasförmige Nebenprodukte, wie z.B. Carbonylsulfid und Methan, sowie höhere Kohlenwasserstoffe werden in den Prozess zurückgeführt. Dies kann sowohl in den Eduktstrom der ersten, zweiten oder dritten Verfahrensstufe erfolgen. Vorteilhafterweise wird vor Rückführung in die zweite oder dritte Verfahrensstufe das CO₂ / CO / H₂ / H₂S-Verhältnis durch Umsetzung mit Wasser auf 1 : 0,1 : 1 : 1 bis 1 : 1 : 10 : 10 eingestellt. Dies kann katalysiert oder nicht-katalysiert in einem Festbettreaktor, einem Reaktionsrohr, einer Waschkolonne oder einer Reaktivdestillation bei einer Temperatur von mindestens 120°C erfolgen. Vorteilhafterweise werden Nebenkomponenten wie z.B. Carbonylsulfid und Schwefelkohlenstoff in diesem Verfahrensschritt zu Kohlendioxid und Schwefelwasserstoff hydrolysiert, welche als Edukte wieder Eingang in den zweiten oder dritten Verfahrensschritt finden. Reaktionskomponenten, wie z.B. Sulfide, Polysulfide und Kohlenwasserstoffe, welche während der Abtrennung von Methylmercaptan im vierten Verfahrensschritt anfallen, können ohne weitere Aufarbeitung in den ersten oder zweiten Verfahrensschritt zurückgeführt werden, wodurch sich die Gesamtselektivität des Prozesses für Methylmercaptan bezogen auf Kohlenstoff auf über 95 % erhöht.

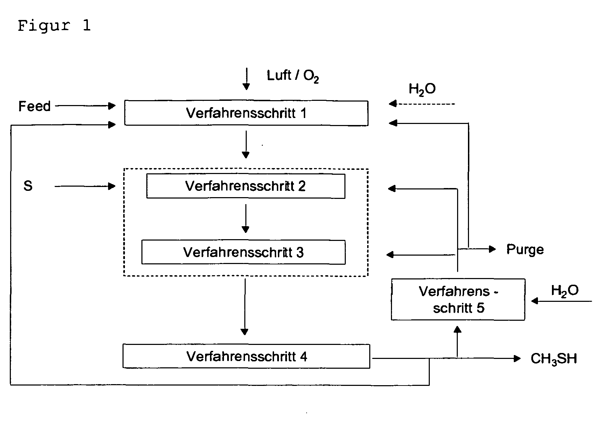

Die Figur 1 dient zur weiteren Erklärung des Verfahrens unter Verwendung der bevorzugt eingesetzten Abfolge von Reaktionsschritten. Wichtig für die Wirtschaftlichkeit des Verfahrens ist die Möglichkeit eine Vielzahl von festen, flüssigen und oder gasförmigen, kohlenstoff- und wasserstoffhaltigen Ausgangsstoffen zu verwenden, die in den ersten Verfahrensschritt eindosiert werden und dass dieser Stoffstrom nicht aufwendig gereinigt und entschwefelt werden muss. Ferner können sämtliche Nebenprodukte, die im vierten Verfahrensschritt abgetrennt werden in den ersten, zweiten oder dritten Verfahrensschritt recycliert werden. Vorteilhafterweise laufen alle Prozessschritte im gleichen Druckbereich ab, so dass auf eine kostenintensive Kompression der Gase zwischen den einzelnen Prozessschritten verzichtet werden kann. Die Umsetzungen erfolgen bei dem Ausgangsdruck der Gase, die den ersten Verfahrensschritt verlassen oder dem zweiten oder dritten Verfahrensschritt aus anderen Quellen unter Prozessdruck zudosiert werden. Vorteilhafterweise wird dieser Druck auf 8 bis 40 bar eingestellt. Im Sinne des Prozesses inerte Gase werden kontinuierlich oder diskontinuierlich über einen Purgegasstrom aus dem Prozess ausgeschleust.

### Beispiel 1:

Durch Dampfreformierung eines Kohlenwasserstoff-Feedgasstroms und anschließende Umsetzung mit Schwefel wurde eine Gasmischung gewonnen, die die Hauptkomponenten CO₂/CO / H₂ / H₂S im Verhältnis von ∼ 1 / 0,1 / 4 / 4 enthält. Bei einer Reaktionstemperatur von 250-350°C und einem Druck von 30 bar wurden die Reaktanden im dritten Verfahrensschritt über verschiedenen Katalysatoren umgesetzt. Die katalytische Aktivität wurde zunächst für einen Durchgang durch den Rektor (Single Pass) bestimmt.

**Tabelle 1**

| Katalysator | Umsatz (CO₂) /% | Selektivität (MC) / | Selektivität (CO) / % | Selektivität (CH₄) / % |
|---|---|---|---|---|
| Katalysator A | 55,8 | 82,2 | 11,8 | 4,5 |
| Re₂O₇/K₂MoO₄ /TiO₂ | | | | |
| Katalysator B | 53,5 | 82,6 | 9,9 | 4,8 |
| SnO₂ / Re₂O₇/K₂MoO₄ /TiO₂ | | | | |
| Katalysator C | 50,1 | 81,5 | 12,6 | 3,5 |
| MnₓO_{Y}/K₂MoO₄ /TiO₂ | | | | |

| | | | | |
|---|---|---|---|---|
| CO₂ = Kohlenstoffdioxid MC = Methylmercaptan | | | | |

### Beispiel 2

Methylmercaptan wurde aus dem Produktgasstrom (Verfahrensschritt 4) von Beispiel 1 abgetrennt. Das Rückgas mit den Hauptkomponenten CO₂, H₂, CO und H₂S wurde nach Umsetzung mit Wasser im Reaktionschritt 5 in den Verfahrensschritt 3 recycliert und unter analogen Reaktionsbedingungen, wie in Beispiel 1 umgesetzt. Es wurden über den Katalysatoren A-C Methylmercaptan-Selektivitäten von 90-94 % erzielt.

## Patentansprüche

1. Verfahren zur kontinuierlichen Herstellung von Methylmercaptan durch Umsetzung eines Eduktgemisches, das kohlenstoff- und wasserstoffhaltige Verbindungen enthält, in einem mehrstufigen Verfahren, bei dem man in Verfahrensschritt 1 die kohlenstoff- und wasserstoffhaltigen Verbindungen mit Luft oder Sauerstoff, gegebenenfalls mit Wasser, zu einer Gasmischung umsetzt, die Kohlendioxid, Kohlenmonoxid und Wasserstoff enthält, in Verfahrensschritt 2 diese Gasmischung mit flüssigem oder gasförmigem Schwefel in einem ein- oder mehrstufigen Verfahrensschritt bei einem Reaktionsdruck von mindestens 5 bar und einer Temperatur von mindestens 200°C umsetzt und das molare CO₂/CO/H₂/H₂S-Verhäl durch Einspeisen von Wasser oder Wasserstoff und gegebenenfalls Schwefelwasserstoff auf ein Verhältnis von 1:0,1:1:0 bis 1:1:10:10 einstellt, in Verfahrensschritt 3 die so erhaltene Gasmischung mit einem Molverhältnis der Verbindungen CO₂ / CO / H₂ / H₂S von 1 : 0,1 : 1 : 1 bis
1 : 1 : 10 : 10 bei einem Reaktionsdruck von mindestens 5 bar und einer Temperatur von mindestens 200°C über einem Katalysator zu einer Reaktionsmischung umsetzt, die als Hauptreaktionsprodukt Methylmercaptan enthält, in Verfahrensschritt 4 dieses Methylmercaptan abtrennt, und in Verfahrensschritt 5 nach Abtrennung der gasförmigen Nebenprodukte die nicht umgesetzten Einsatzstoffe nach optionaler Umsetzung mit Wasser in den Prozess zurückführt, wobei die Gasmischung den ersten Verfahrensschritt bei einem Druck von mindestens 5 bar verlässt und direkt ohne weitere Kompression dem zweiten, und in der Folge die entsprechenden Gasmischungen bei diesem Druck dem dritten und vierten Verfahrensschritt zugeführt werden.

2. Verfahren gemäß Anspruch 1, wobei das Eduktgemisch stickstoffhaltige Verbindungen enthält.

3. Verfahren gemäß den Ansprüchen 1 oder 2, wobei das Eduktgemisch weitere schwefelhaltige Verbindungen enthält.

4. Verfahren gemäß den Ansprüchen 1 bis 3, bei dem das Eduktgemisch weitere Verbindungen enthält, die kohlenstoff-, wasserstoff- und sauerstoffhaltig sind.

5. Verfahren gemäß den Ansprüchen 1 bis 4, bei dem man die kohlenstoff- und wasserstoffhaltigen und gegebenenfalls sauerstoffhaltigen Verbindungen durch partielle Oxidation mit Luft oder Sauerstoff oder durch Dampfreformierung in Gasmischungen überführt, die CO₂, CO, H₂ und optional H₂S enthalten.

6. Verfahren gemäß den Ansprüchen 1 bis 5 bei dem die im Eduktgemisch enthaltenen Verbindungen aus Abgasströmen von Prozessen zur Erzeugung von Energie oder chemischen Produkten stammen.

7. Verfahren nach Anspruch 6, bei dem man Stoffströme aus der Aufarbeitung von Prozessen zur Oxidation von Kohlenwasserstoffen und zur Synthese von Stickstoff- und Schwefelhaltigen Verbindungen als Rohstoffe dem Prozess zuführt.

8. Verfahren nach Anspruch 1 bis 6 wobei in biologischen Stoffwechselprozessen erzeugte Gasmischungen in den Verfahrensschritten 2 oder 3 eingeleitet werden.

9. Verfahren nach den Ansprüchen 6, 7 oder 8 bei dem die Gasmischungen mindestens eine Verbindung, ausgewählt aus der Gruppe Methan, höhere Kohlenwasserstoffe mit C₂-C₆-Rest, CO₂ und CO enthält.

10. Verfahren gemäß den Ansprüchen 1 bis 3, bei dem im Verfahrenschritt 2 die Umsetzung zu einer Gasmischung, die CO₂, CO, H₂ und H₂S enthält, in Gegenwart von flüssigem oder gasförmigem Schwefel in einer ein- oder mehrstufigen nichtkatalysierten Homogenreaktion oder unter Verwendung eines Katalysators erfolgt.

11. Verfahren gemäß den Ansprüchen 1 bis 10 **dadurch gekennzeichnet, dass** das CO₂ / CO / H₂ / H₂S Verhältnis am Ende des Verfahrensschrittes 2 von 1 : 0,1 : 1 : 1 bis
1 : 0,1 : 4 : 4 reicht.

12. Verfahren gemäß den Ansprüchen 1 bis 11, bei dem man das Methylmercaptan aus dem Reaktionsgas abtrennt und nicht umgesetzte gasförmige Einsatzstoffe in die Verfahrensschritte 1, 2 oder 3 zurückführt.

13. Verfahren gemäß den Ansprüchen 1 bis 12, bei dem man die Gesamtmenge des Schwefelwasserstoffs im Verfahrensschritt 3 durch Variation des Kohlenstoff-Wasserstoffverhältnises der im Eduktgemisch enthaltenen Verbindungen bzw. des H₂-Anteils im Reaktionsgas, das man in dem Verfahrensschritt 2 zuführt und durch Variation eines oder mehrerer der Verfahrensparameter, ausgewählt aus der Gruppe: Verweilzeit, Reaktionstemperatur und Reaktionsdruck einstellt.

14. Verfahren nach den Ansprüchen 1 bis 4 bei dem man in den Verfahrensschritten 2 und 3 Reaktivdestillationen, Blasensäulen-, Festbett-, Horden- oder Rohrbündelreaktoren für die katalysierte Umsetzung zu Methylmercaptan einsetzt.

15. Verfahren nach den Ansprüchen 1 bis 5 bei dem man die Verfahrensschritte 2 und 3 in einem Reaktionsapparat kombiniert.

16. Verfahren gemäß den Ansprüchen 1 bis 15 bei dem man Katalysatoren einsetzt, die Übergangsmetalloxide oder -sulfide als Promotoren enthaltende Alkaliwolframate, Alkalimolybdate oder halogenidhaltige Alkaliwolframate oder Alkalimolybdate enthalten.

17. Verfahren gemäß den Ansprüchen 1 bis 16, bei dem die Umsetzung der Gasmischung in Verfahrensschritt 3 über Übergangsmetall- und Alkalimetalloxide oder - sulfide als Promotoren enthaltenden Molybdat- oder Wolframat-haltigen Katalysatoren erfolgt.

18. Verfahren gemäß Anspruch 16, bei dem man mindestens einen der Promotoren ausgewählt aus der Gruppe Cobalt-, Mangan- und Rhenium-Oxide oder Sulfide enthaltende Alkaliwolframate, Alkalimolybdate oder halogenidhaltige Alkaliwolframate oder Alkalimolybdate als Katalysatoren einsetzt.

19. Verfahren gemäß den Ansprüchen 1 bis 15, bei dem man Trägerkatalysatoren einsetzt, die oxidische Mo- und K-Verbindungen enthalten, wobei Mo und K in einer Verbindung enthalten sein können und die mindestens eine aktive oxidische Verbindung der allgemeinen Formel AₓO_{y} enthalten, A ein Element der Mangangruppe, insbesondere Mn oder Re, und x und y ganze Zahlen von 1 bis 7 bedeuten.

20. Verfahren gemäß den Ansprüchen 1 bis 16 und 19 bei dem die Katalysatoren die Verbindungen bevorzugt in einem Gewichtsverhältnis von
AₓO_{y}/K₂MoO₄/Träger = (0,001-0,5)/(0,01-0,8)/1 bzw.
AₓO₄/MoO₃/K₂O/Träger = [0,001-0,5)/(0,01-0,8)/0,005-0,5)/1
enthalten.

21. Verfahren gemäß den Ansprüchen 1 bis 18 und 20, wobei die Katalysatoren einen oder mehrere Promotoren enthalten, ausgewählt aus der Gruppe der oxidischen Verbindungen mit der allgemeinen Formel MₓO_{y}, in denen M für ein Übergangselement oder ein Metall aus der Gruppe der Seltenen Erden steht und x und y eine ganze Zahl von 1 bis 7 bedeuten.

22. Verfahren gemäß den Ansprüchen 1 bis 16 und 20 bis 21, wobei die Verhältnisse der Gewichtsanteile in den Bereichen liegen:
K₂MoO₄/MₓO_{y}/Träger = (0,01-0,80)/(0,01-0,1)/1,
MoO₃/K₂O/MₓO_{y}/Träger = [0,10-0,50)/(0,10-0,30)/(0,01-0,1)/1
wobei x und y eine ganze Zahl von 1 bis 7 bedeuten.

## Claims

1. Process for continuously preparing methyl mercaptan by converting a reactant mixture which comprises carbon- and hydrogen-containing compounds in a multistage process in which the carbon- and hydrogen-containing compounds are reacted in process step 1 with air or oxygen, optionally with water, to give a gas mixture which comprises carbon dioxide, carbon monoxide and hydrogen, this gas mixture is reacted in process step 2 with liquid or gaseous sulphur in a one-stage or multistage process step at a reaction pressure of at least 5 bar and a temperature of at least 200°C, and the molar CO₂/CO/H₂/H₂S ratio is adjusted to a ratio of 1:0.1:1:0 to 1:1:10:10 by feeding in water or hydrogen and optionally hydrogen sulphide, the gas mixture thus obtained having a molar ratio of the compounds CO₂/CO/H₂/H₂S of 1:0.1:1:1 to 1:1:10:10 is converted in process step 3 at a reaction pressure of at least 5 bar and a temperature of at least 200°C over a catalyst to a reaction mixture which comprises methyl mercaptan as the main reaction product, this methyl mercaptan is removed in process step 4, and, in process step 5, after removal of the gaseous by-products, the unconverted feedstocks, after optional reaction with water, are recycled into the process, wherein the gas mixture leaves the first process step at a pressure of at least 5 bar and is fed directly, without further compression, to the second process step, and the corresponding gas mixtures are subsequently fed at this pressure to the third and fourth process steps.

2. Process according to Claim 1, wherein the reactant mixture comprises nitrogen-containing compounds.

3. Process according to Claim 1 or 2, wherein the reactant mixture comprises further sulphur-containing compounds.

4. Process according to Claims 1 to 3, in which the reactant mixture comprises further compounds which are carbon-, hydrogen- and oxygen-containing.

5. Process according to Claims 1 to 4, in which the carbon- and hydrogen-containing and optionally oxygen-containing compounds are converted by partial oxidation with air or oxygen or by steam reformation to gas mixtures which comprise CO₂, CO, H₂ and optionally H₂S.

6. Process according to Claims 1 to 5, in which the compounds present in the reactant mixture stem from offgas streams of processes for generating energy or chemical products.

7. Process according to Claim 6, in which streams from the workup of processes for oxidation of hydrocarbons and for synthesis of nitrogen- and sulphur-containing compounds are supplied to the process as raw materials.

8. Process according to Claims 1 to 6, wherein gas mixtures generated in biological metabolism processes are introduced into process steps 2 or 3.

9. Process according to Claim 6, 7 or 8, in which the gas mixtures comprise at least one compound selected from the group of methane, higher hydrocarbons having a C₂-C₆ radical, CO₂ and CO.

10. Process according to Claims 1 to 3, in which the conversion to a gas mixture which comprises CO₂, CO, H₂ and H₂S in process step 2 is effected in the presence of liquid or gaseous sulphur in a one-stage or multistage uncatalysed homogeneous reaction or using a catalyst.

11. Process according to Claims 1 to 10, **characterized in that** the CO₂/CO/H₂/H₂S ratio at the end of process step 2 ranges from 1:0.1:1:1 to 1:0.1:4:4.

12. Process according to Claims 1 to 11, in which the methyl mercaptan is removed from the reaction gas and unconverted gaseous feedstocks are recycled into process steps 1, 2 or 3.

13. Process according to Claims 1 to 12, in which the total amount of hydrogen sulphide in process step 3 is established by varying the carbon-hydrogen ratio of the compounds present in the reactant mixture or the H₂ content in the reaction gas which is fed into process step 2, and by varying one or more process parameters selected from the group of: residence time, reaction temperature and reaction pressure.

14. Process according to Claims 1 to 4, in which, in process steps 2 and 3, reactive distillations, bubble column reactors, fixed bed reactors, staged reactors or tube bundle reactors are used for the catalysed conversion to methyl mercaptan.

15. Process according to Claims 1 to 5, in which process steps 2 and 3 are combined in one reaction apparatus.

16. Process according to Claims 1 to 15, in which catalysts which comprise alkali metal tungstates, alkali metal molybdates or halide-containing alkali metal tungstates or alkali metal molybdates which comprise transition metal oxides or sulphides as promoters are used.

17. Process according to Claims 1 to 16, in which the gas mixture is converted in process step 3 over molybdate- or tungstate-containing catalysts which comprise transition metal oxides or sulphides and alkali metal oxides or sulphides as promoters.

18. Process according to Claim 16, in which at least one of the promoters selected from the group of alkali metal tungstates, alkali metal molybdates or halide-containing alkali metal tungstates or alkali metal molybdates which comprise cobalt oxides or sulphides, manganese oxides or sulphides and rhenium oxides or sulphides is used as a catalyst.

19. Process according to Claims 1 to 15, in which supported catalysts which comprise oxidic Mo and K compounds are used, where Mo and K may be present in one compound, and which comprise at least one active oxidic compound of the general formula AₓO_{y}, A is an element of the manganese group, especially Mn or Re, and x and y are integers of 1 to 7.

20. Process according to Claims 1 to 16 and 19, in which the catalysts comprise the compounds preferably in a weight ratio of
AₓO_{y}/K₂MoO₄/support = (0.001-0.5)/(0.01-0.8)/1
or
AₓO₄/MoO₃/K₂O/support = (0.001-0.5)/(0.01-0.8)/ / (0.005-0.5) /1.

21. Process according to Claims 1 to 18 and 20, wherein the catalysts comprise one or more promoters selected from the group of the oxidic compounds with the general formula MₓO_{y} in which M is a transition element or a metal from the group of the rare earths, and x and y are each an integer of 1 to 7.

22. Process according to Claims 1 to 16 and 20 to 21, wherein the ratios of the proportions by weight are within the ranges of:
K₂MoO₄/MₓO_{y}/support = (0.01-0.80)/(0.01-0.1)/1,
MoO₃/K₂O/MₓO_{y}/support = (0.10-0.50)/(0.10-0.30)/ (0.01-0.1)/1,
where x and y are each an integer of 1 to 7.

## Revendications

1. Procédé de fabrication continue de méthylmercaptan par mise en réaction d'un mélange de réactifs contenant des composés contenant du carbone et de l'hydrogène par un procédé à plusieurs étapes, selon lequel, à l'étape de procédé 1, les composés contenant du carbone et de l'hydrogène sont mis en réaction avec de l'air ou de l'oxygène, éventuellement avec de l'eau, pour former un mélange gazeux contenant du dioxyde de carbone, du monoxyde de carbone et de l'hydrogène, à l'étape de procédé 2, ce mélange gazeux est mis en réaction avec du soufre liquide ou gazeux par une étape de procédé à une ou plusieurs étapes à une pression de réaction d'au moins 5 bar et à une température d'au moins 200 °C, et le rapport molaire CO₂/CO/H₂/H₂S est ajusté à un rapport de 1:0,1:1:0 à 1:1:10:10 par introduction d'eau ou d'hydrogène et éventuellement de sulfure d'hydrogène, à l'étape de procédé 3, le mélange gazeux ainsi obtenu présentant un rapport molaire entre les composés CO₂/CO/H₂/H₂S de 1:0,1:1:1 à 1:1:10:10 est mis en réaction à une pression de réaction d'au moins 5 bar et à une température d'au moins 200 °C sur un catalyseur pour former un mélange réactionnel contenant en tant que produit de réaction principal du méthylmercaptan, à l'étape de procédé 4, ce méthylmercaptan est séparé, et à l'étape de procédé 5, après la séparation des produits secondaires gazeux, les matières premières non réagies sont recyclées dans le procédé après mise en réaction optionnelle avec de l'eau, dans lequel le mélange gazeux quitte la première étape de procédé à une pression d'au moins 5 bar et est introduit directement sans compression supplémentaire dans la deuxième, et les mélanges gazeux correspondants sont en conséquence introduits à cette pression dans la troisième et la quatrième étape de procédé.

2. Procédé selon la revendication 1, dans lequel le mélange de réactifs contient des composés azotés.

3. Procédé selon les revendications 1 ou 2, dans lequel le mélange de réactifs contient d'autres composés soufrés.

4. Procédé selon les revendications 1 à 3, dans lequel le mélange de réactifs contient d'autres composés contenant du carbone, de l'hydrogène et de l'oxygène.

5. Procédé selon les revendications 1 à 4, dans lequel les composés contenant du carbone et de l'hydrogène et éventuellement de l'oxygène sont transformés par oxydation partielle avec de l'air ou de l'oxygène ou par reformage à la vapeur en mélanges gazeux contenant CO₂, CO, H₂ et éventuellement H₂S.

6. Procédé selon les revendications 1 à 5, dans lequel les composés contenus dans le mélange de réactifs sont issus de courants de gaz d'échappement de procédés de génération d'énergie ou de produits chimiques.

7. Procédé selon la revendication 6, dans lequel des courants de matière issus du traitement de procédés pour l'oxydation d'hydrocarbures et pour la synthèse de composés azotés et soufrés sont introduits dans le procédé en tant que matières premières.

8. Procédé selon les revendications 1 à 6, dans lequel des mélanges gazeux formés lors de procédés métaboliques biologiques sont introduits dans les étapes de procédé 2 ou 3.

9. Procédé selon les revendications 6, 7 ou 8, dans lequel les mélanges gazeux contiennent au moins un composé choisi dans le groupe constitué par le méthane, les hydrocarbures supérieurs contenant un radical en C₂-C₆, CO₂ et CO.

10. Procédé selon les revendications 1 à 3, dans lequel la réaction à l'étape de procédé 2 pour former un mélange gazeux contenant CO₂, CO, H₂ et H₂S a lieu en présence de soufre liquide ou gazeux par une réaction homogène non catalysée à une ou plusieurs étapes ou en utilisant un catalyseur.

11. Procédé selon les revendications 1 à 10, **caractérisé en ce que** le rapport CO₂/CO/H₂/H₂S à la fin de l'étape de procédé 2 atteint 1:0,1:1:1 à 1:0,1:4:4.

12. Procédé selon les revendications 1 à 11, dans lequel le méthylmercaptan est séparé du gaz réactionnel et les matières premières gazeuses non réagies sont recyclées dans les étapes de procédé 1, 2 ou 3.

13. Procédé selon les revendications 1 à 12, dans lequel la quantité totale de sulfure d'hydrogène à l'étape de procédé 3 est ajustée par variation du rapport carbone-hydrogène des composés contenus dans le mélange de réactifs ou de la proportion d'H₂ dans le gaz réactionnel introduit dans l'étape de procédé 2 et par variation d'un ou de plusieurs des paramètres de procédé choisi dans le groupe : temps de séjour, température de réaction et pression de réaction.

14. Procédé selon les revendications 1 à 4, dans lequel des distillations réactives, des réacteurs à colonne à bulles, à lit fixe, à plateaux ou à faisceau de tubes sont utilisés pour la réaction catalysée en méthylmercaptan aux étapes de procédé 2 et 3.

15. Procédé selon les revendications 1 à 5, dans lequel les étapes de procédé 2 et 3 sont combinées dans un appareil de réaction.

16. Procédé selon les revendications 1 à 15, dans lequel des catalyseurs sont utilisés, qui contiennent des tungstates alcalins, des molybdates alcalins ou des tungstates alcalins ou molybdates alcalins contenant des halogénures, contenant des oxydes ou sulfures de métaux de transition en tant que promoteurs.

17. Procédé selon les revendications 1 à 16, dans lequel la réaction du mélange gazeux à l'étape de procédé 3 a lieu sur des catalyseurs contenant du molybdate ou du tungstate, contenant des oxydes ou sulfures de métaux de transition et de métaux alcalins en tant que promoteurs.

18. Procédé selon la revendication 16, dans lequel des tungstates alcalins, des molybdates alcalins ou des tungstates alcalins ou molybdates alcalins contenant des halogénures, contenant au moins un des promoteurs choisi dans le groupe constitué par les oxydes ou sulfures de cobalt, de manganèse et de rhénium, sont utilisés en tant que catalyseurs.

19. Procédé selon les revendications 1 à 15, dans lequel des catalyseurs supportés sont utilisés, qui contiennent des composés oxydiques de Mo et de K, Mo et K pouvant être contenus dans un composé, et qui contiennent au moins un composé oxydique actif de formule générale AₓO_{y}, A signifiant un élément du groupe du manganèse, notamment Mn ou Re, et x et y signifiant des nombres entiers de 1 à 7.

20. Procédé selon les revendications 1 à 16 et 19, dans lequel les catalyseurs contiennent de préférence les composés en un rapport en poids de
AₓO_{y}/K₂MoO₄/support = (0,001-0,5)/(0,01-0,8)/1
ou
AₓO₄/MoO₃/K₂O/support = (0,001-0,5)/(0,01-0,8)/(0,005-0,5)/1.

21. Procédé selon les revendications 1 à 18 et 20, dans lequel les catalyseurs contiennent un ou plusieurs promoteurs choisis dans le groupe des composés oxydiques de formule générale MₓO_{y}, M représentant un élément de transition ou un métal du groupe des terres rares et x et y signifiant un nombre entier de 1 à 7.

22. Procédé selon les revendications 1 à 16 et 20 à 21, dans lequel les rapports entre les proportions en poids se situent dans les plages :
K₂MoO₄/MₓO_{y}/support = (0,01-0,80)/(0,01-0,1)/1,
MoO₃/K₂O/MₓO_{y}/support = (0,10-0,50)/(0,10-0,30)/(0,01-0,1)/1,
x et y signifiant un nombre entier de 1 à 7.
